# EUROPEAN PATENT SPECIFICATION

(11) **EP 2 408 519 B1**
(45) Date of publication and mention of the grant of the patent: **31.08.2016**
(21) Application number: 10752992.7
(22) Date of filing: 15.03.2010
(51) Int. Cl.: A61N 1/36, H04L 27/12, H04R 25/00

(54) **TRANSCUTANEOUS MODULATED POWER LINK FOR A MEDICAL IMPLANT**
TRANSKUTANE MODULIERTE LEISTUNGSVERKNÜPFUNG FÜR EIN MEDIZINISCHES IMPLANTAT
LIAISON ÉLECTRIQUE TRANSCUTANÉE MODULÉE POUR IMPLANT MÉDICAL

(30) Priority: 16.03.2009 AU 2009901122
(43) Date of publication of application: 25.01.2012
(73) Proprietor: Cochlear Limited, Macquarie University, NSW 2109 (AU)
(72) Inventor: MESKENS, Werner, Lane Cove NSW 2066 (AU)
(74) Representative: Grünecker Patent- und Rechtsanwälte PartG mbB
(86) International application number: PCT/AU2010/000300
(87) International publication number: WO 2010/105291

(56) References cited:
- AU-A1- 2008 201 690
- DE-B1- 2 823 798
- US-A- 4 408 608
- US-A- 5 825 257
- US-A- 6 073 050
- US-B1- 6 219 580
- CLEMENTS M. ET AL.: 'An Implantable Neuro-Stimulator Device tor a Ketmal Prosthesis' IEEE INTERNATIONAL SOLID-STATE CIRCUITS CONFERENCE, 1999, DIGESTIF TECHNICAL PAPERS 1999, pages 216 - 217, XP032387460
- KAWAHITO, S. ET AL.: 'A CMOS Integrated Circuit for Multichannel Multiple-Subject Biotelemetry Using Bidirectional Optical Transmissions' IEEE TRANSACTIONS ON BIOMEDICAL ENGINEERING vol. 41, no. 4, 1994, pages 400 - 406, XP055100296
- MANDAL ET AL.: 'A Bidirectional Wireless Link for Neural Prostheses that Minimizes Implanted Power Consumption' IEEE BIOMEDICAL CIRCUITS AND SYSTEMS CONFERENCE, 2007. BIOCAS 2007. 2007, pages 45 - 48, XP031237028

## Description

### FIELD

The present invention relates to a modulated power link for use with an implanted mechanical actuator.

The following documents are referred to in the present description:
- US Patent No. 6,240,318 entitled "Transcutaneous Energy Transmission System With Full Wave Class E Rectifier"
- International Patent Application No. PCT/AU2005/001801 (WO2006/058368) entitled "Implantable Actuator For Hearing Aid Applications"

### BACKGROUND

A variety of medical implants exists to assist users who suffer from a loss of one or more senses, such as sight or hearing.

Users who suffer from a loss of hearing may be assisted by various devices. One such device is a hearing aid, which amplifies and/or clarifies surrounding sounds and directs this into the user's ear. Another device is a cochlear implant, which provides stimulating electrical energy directly to the user's auditory nerves in the cochlea. Another type of hearing device which may be used if the user's cochlea is functioning well, but the middle ear is defective, is a mechanical actuator which provides direct mechanical vibrations to a part of the user's hearing system such as the middle ear, inner ear, or bone surrounding the hearing system. One example of such a device is known as a Direct Acoustic Cochlear Stimulation (DACS) system, in which the actuator operates directly on the cochlea.

A DACS system consists of an external part which receives and processes surrounding acoustic energy, to control an internal part, including the actuator. The external part receives and processes the acoustic energy into data and converts this into signals that can be transmitted wirelessly through the skin of the user via a coil transmitter in the external part. The internal, implanted part has a receive coil for receiving the transmitted data and converting this into control signals that control the movement of the actuator, which acts directly onto a part of the user's hearing system such as a part of the inner ear (e.g. the stapes) or directly onto the oval window of the cochlea. This then generates vibrations on the cochlea fluid which stimulates hair cells which then stimulates nerves connected directly to the brain to be perceived as sound, as is the normal function of the cochlea.

As will be appreciated, the internal part requires power to operate. In some types of DACS systems, the power is provided by a local power supply, however, in some systems, the power may be provided via a transcutaneous power link transmitted and received through the external and internal coils respectively.

DE 2823798 relates to a method for electrically stimulating the auditory nerves and also relates to multichannel hearing prosthesis for carrying out this method. More particularly, this document discloses a method for generating electrical signals for directly stimulating the auditory nerves through a Cochlear implant.

### SUMMARY

In one aspect, a method is disclosed of providing a control signal to an actuator of an implanted medical device as set forth in claim 1. The method comprises receiving a transmission signal comprising a pulse modulated electrical signal further modulated using digital modulation, digitally demodulating the received transmission signal to remove the digital modulation and then applying the demodulated signal to an input of an amplifier to provide the control signal for the actuator.

In another aspect, an internal module for a medical implant system is disclosed as set forth in claim 6. The internal module comprises a receiver for receiving a transmission signal comprising a pulse modulated electrical signal further modulated using digital modulation, a digital demodulator for digitally demodulating the received transmission signal and an amplifier for receiving the digitally demodulated signal and for providing a control signal to an actuator.

In a further aspect, a Direct Acoustic Cochlear Stimulator system is disclosed as set forth in claim 11. The Direct Acoustic Cochlear Stimulator comprises an external module comprising an input for receiving an external signal and for converting the received external signal into an electrical signal, a pulse modulator for modulating the electrical signal to provide a pulse modulated signal, a digital modulator for modulating the pulse modulated signal to provide a transmission signal and an antenna for transmitting the transmission signal. The system also comprises an internal module comprising a receiver for receiving the transmission signal, a digital demodulator for digitally demodulating the received transmission signal and an amplifier for receiving the digitally demodulated signal and for providing a control signal to an actuator. Moreover, the external module of a medical implant system is disclosed for processing an external signal for transmitting transcutaneously to an implanted internal module of the medical implant system. The external module comprises an input for receiving the external signal and for converting the received external signal into an electrical signal, a pulse modulator for modulating the electrical signal to provide a pulse modulated signal, a digital modulator for modulating the pulse modulated signal to provide a transmission signal and then an antenna for transmitting the transmission signal.

### DRAWINGS

Figure 1 - shows one example of a Direct Acoustic Cochlear Stimulator system to which the various aspects of the present invention may be applied;
Figure 2 - shows a broad method of processing an external signal in medical implant system;
Figure 3 - shows one example implementation of the method of Figure 2 in an external module of the medical implant system;
Figure 4 - shows a broad method of providing a control signal to an actuator of a medical implant;
Figure 5 - shows one example implementation of the method of Figure 4 in an internal module of the medical implant system;
Figure 6 - shows a broad arrangement of a medical implant system incorporating the external module of

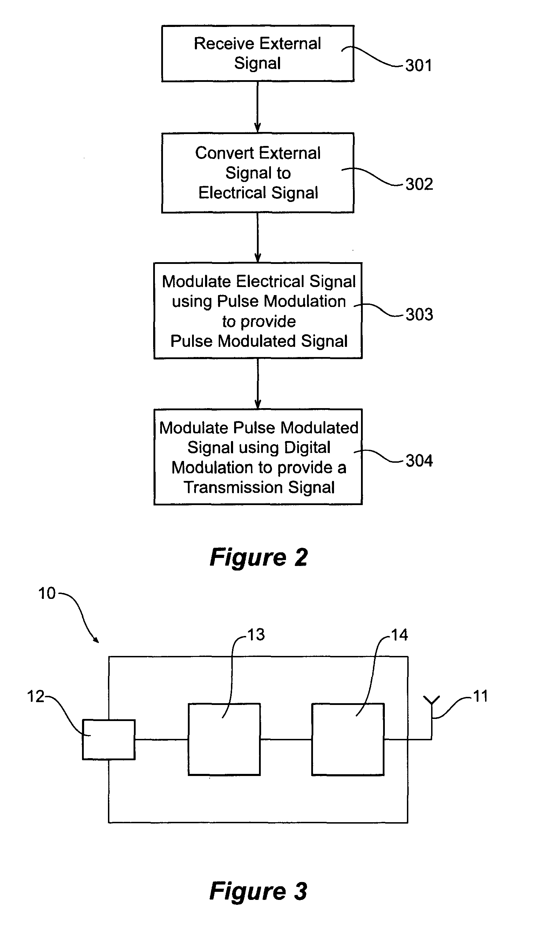

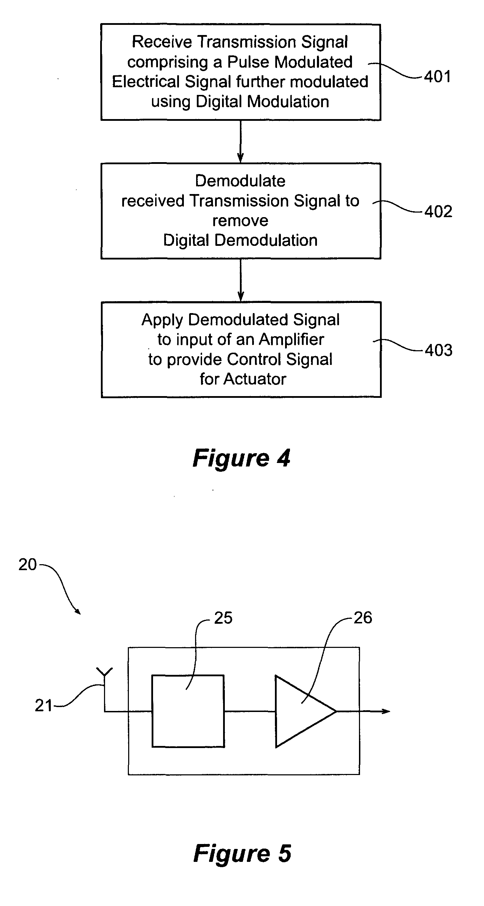

Figure 3 and the internal module of Figure 5;

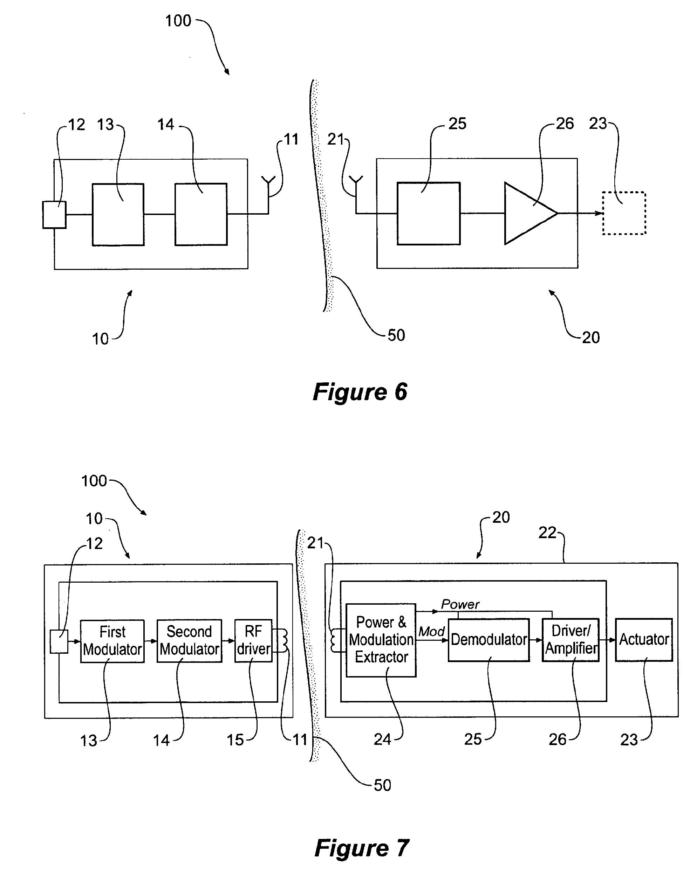

Figure 7 - shows a high-level system block diagram of the components of an example stimulation system;

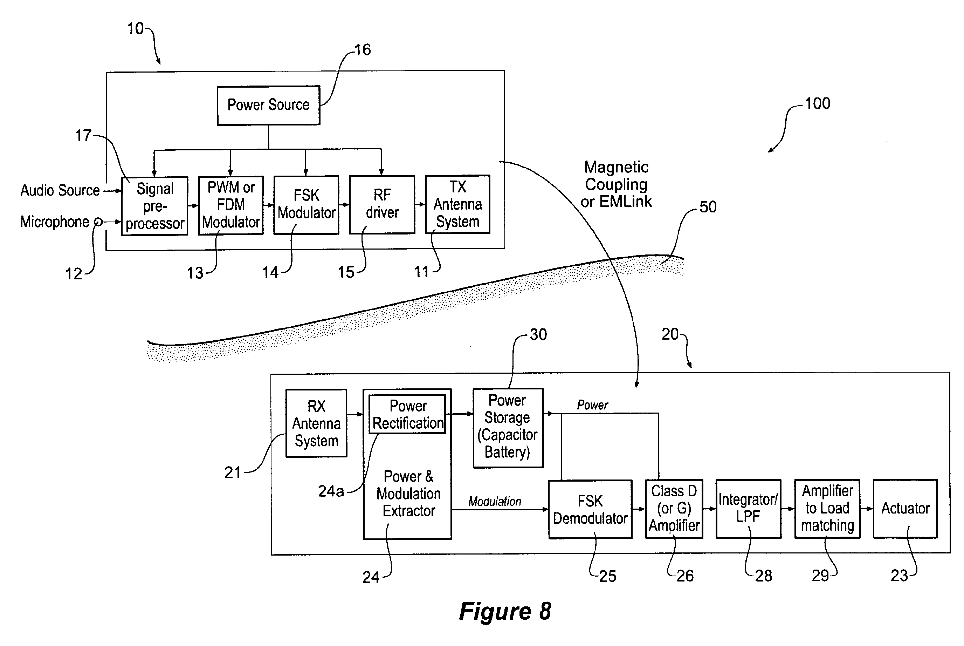

Figure 8 - shows a more detailed system block diagram of an example arrangement of a stimulation system;

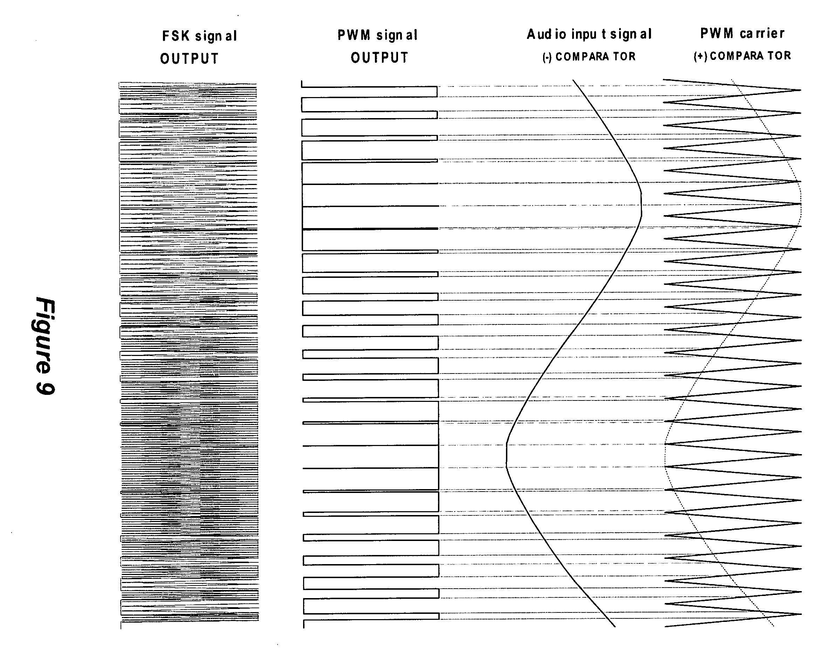

Figure 9 - shows a time diagram of PWM using a triangular wave and FSK generation;

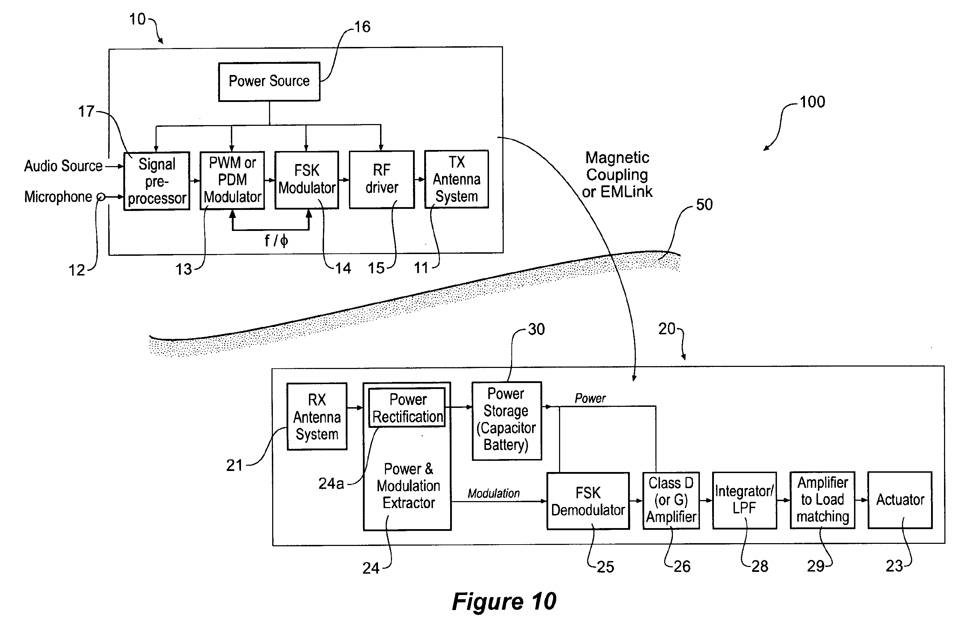

Figure 10 - shows an alternative embodiment of the arrangement of Figure 8;

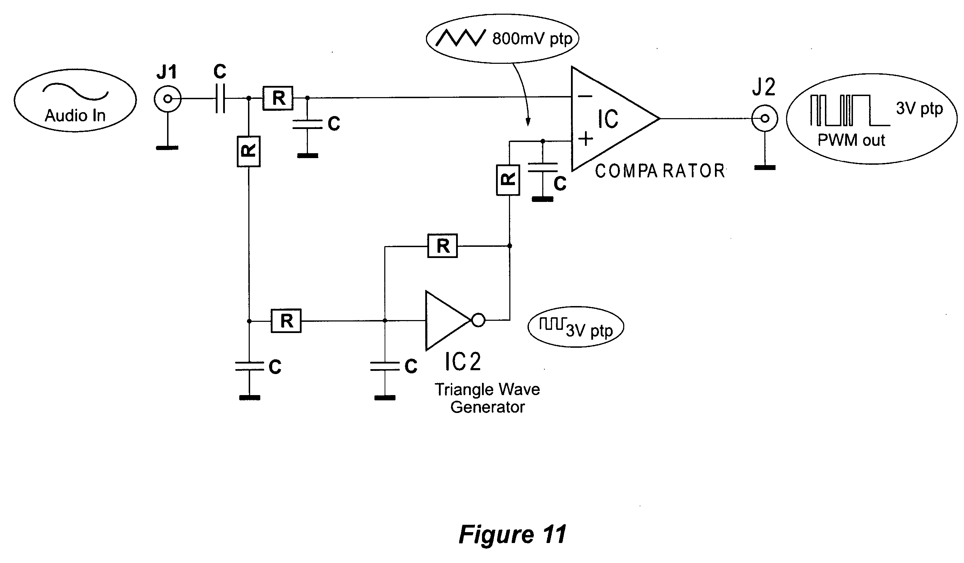

Figure 11 - shows an example of a PWM modulation circuitry;

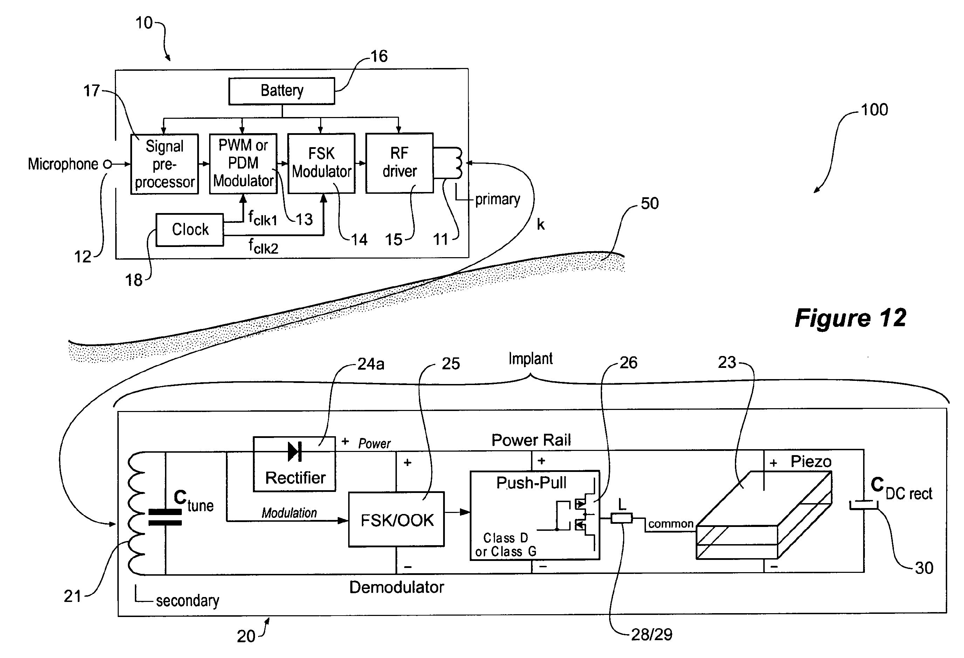

Figure 12 - shows an example of the various aspects of the present invention applied to a T-BAHA system;

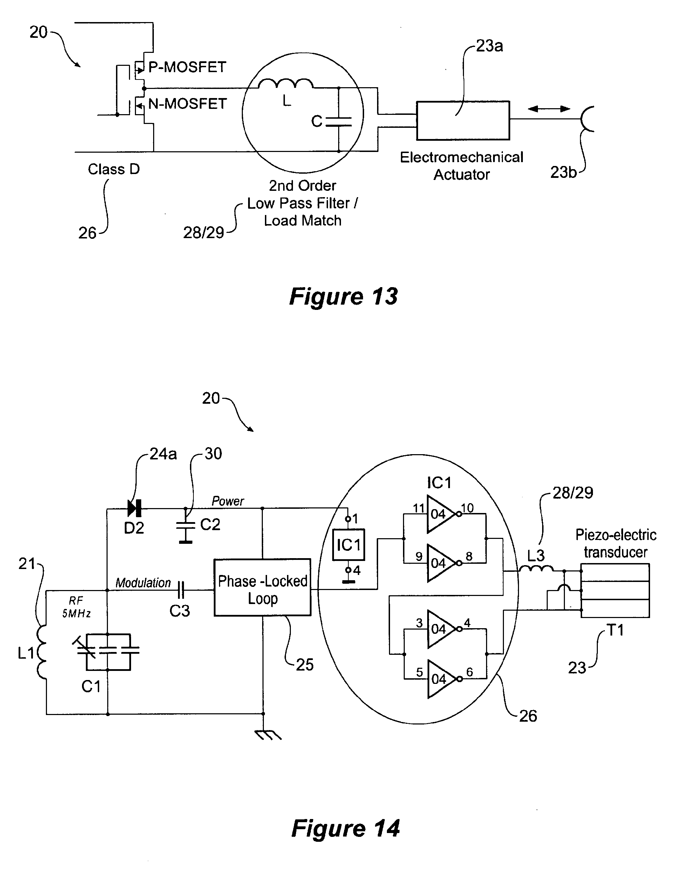

Figure 13 - shows an example of some components in the internal module of a DACS system according to one aspect of the present invention;
Figure 14 - shows a circuit diagram of an internal part with FSK demodulation by a phase-locked loop (PLL);

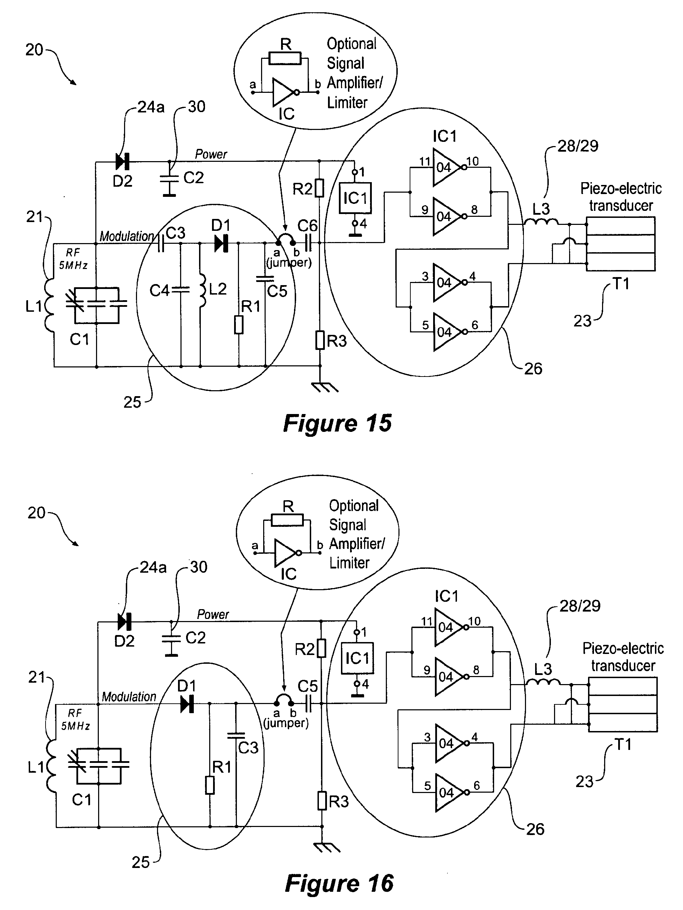

Figure 15 - shows a circuit diagram of an internal part with FSK demodulation using slope detection;
Figure 16 - shows a circuit diagram of an internal part with FSK demodulation using slope detection; a

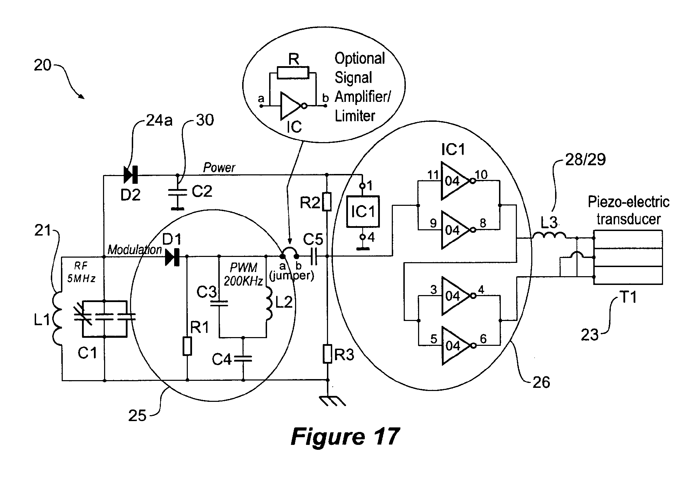

Figure 17 - shows an alternative embodiment to the arrangement of Figure 16;

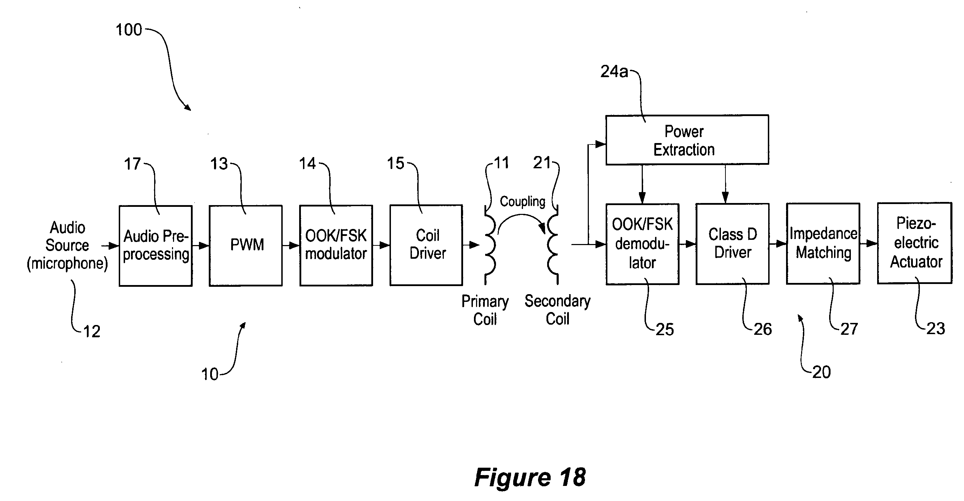

Figure 18 - shows a block diagram of ne specific embodiment of a medical implant system;

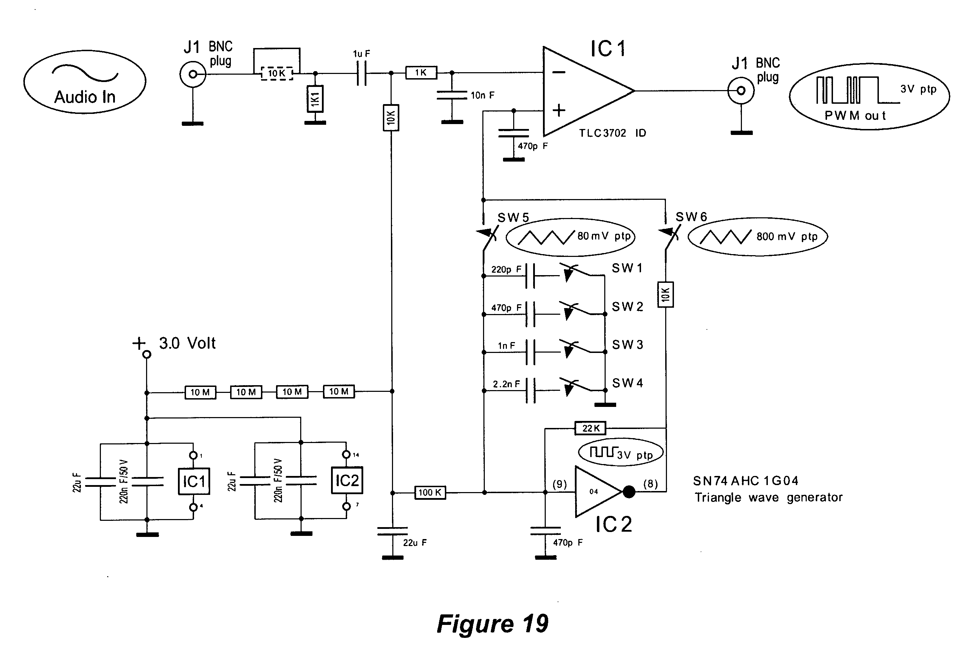

Figure 19 - shows an example circuit arrangement of the PWM of Figure 18;

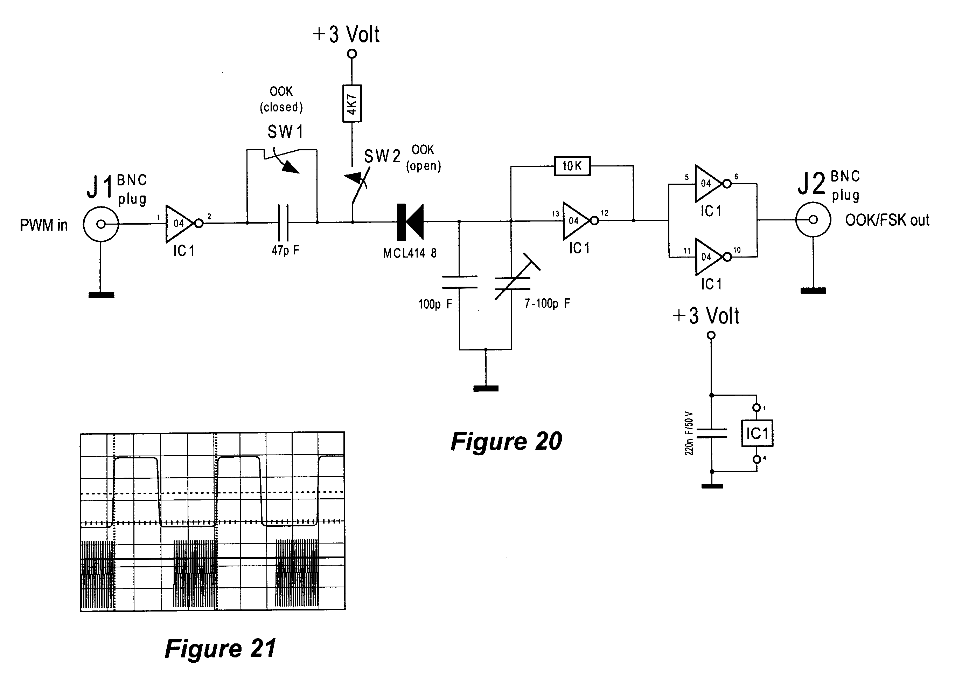

Figure 20 - shows an example circuit arrangement of the OOK modulator of Figure 18;
Figure 21 - shows the input and outputs waveforms of the circuit of Figure 20;

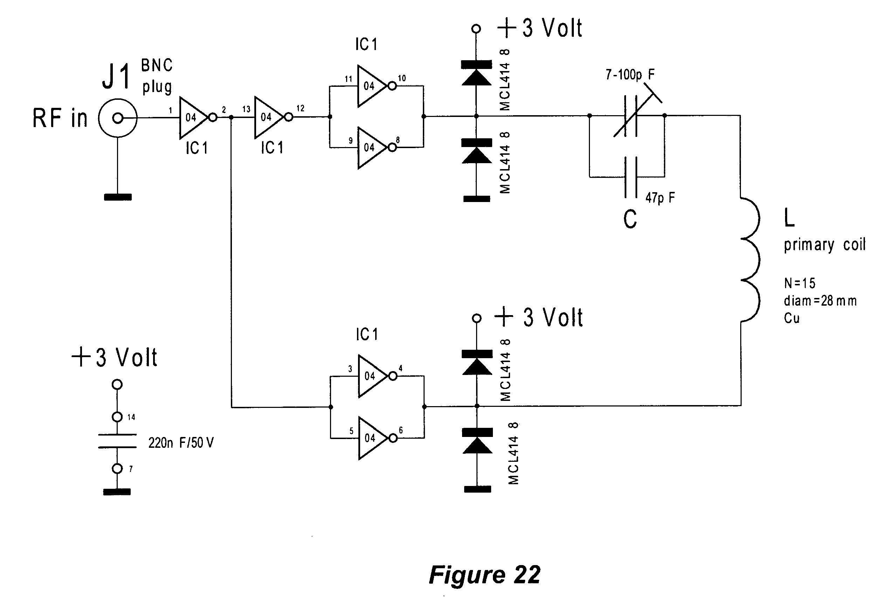

Figure 22 - shows an example circuit arrangement of the coil driver of Figure 18; and

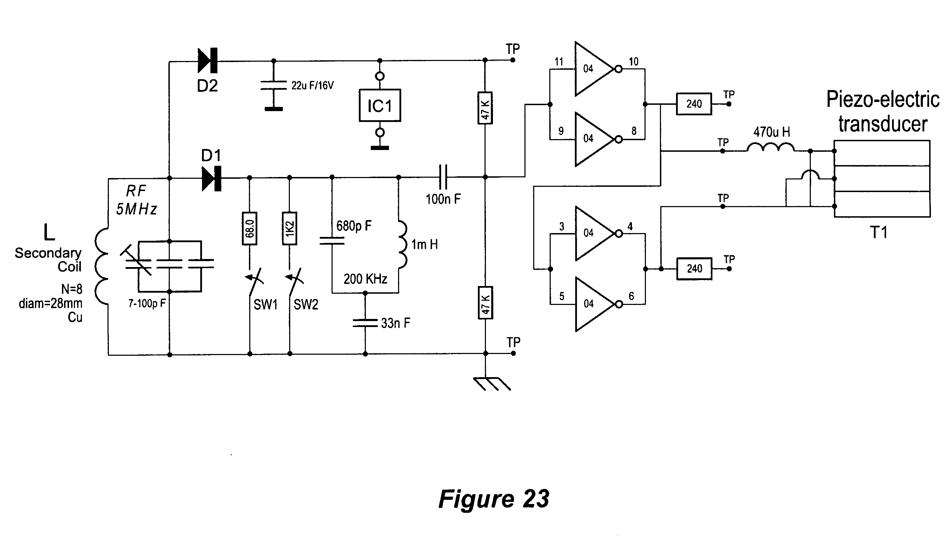

Figure 23 - shows an example circuit arrangement of the internal module of Figure 18.

### DETAILED DESCRIPTION

While the various aspects will largely be described with reference to a DACS system, it will be understood that the various aspects may be applied to any direct electromechanical stimulation implant system including any middle ear, inner ear and transcutaneous bone anchored hearing aid implant systems.

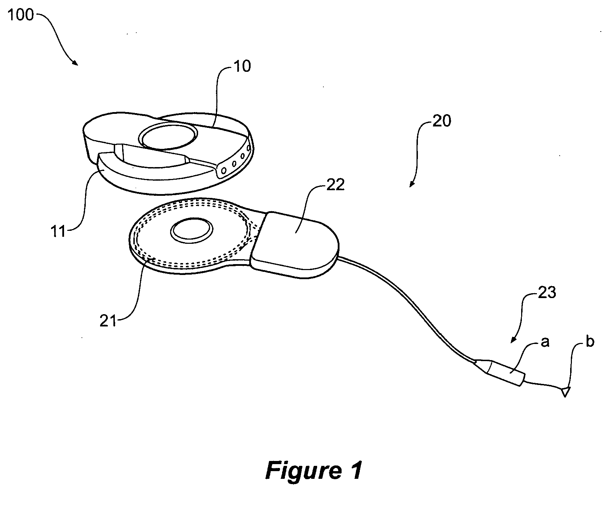

Figure 1 shows an example of a DACS system 100 to which the various aspects described herein may be applied. The system 100 includes an external part 10 and an internal part 20. External part 10 includes a microphone (not shown) or other transducer for detecting and receiving acoustic energy from the environment, a processing system (not shown) inside the casing of the external part, and a wireless antenna such as a coil 11 for transmitting the processed signals wirelessly. The internal part 20, to be implanted in the user, includes an internal antenna or receiving coil 21, receiver and processor electronic circuits embedded in an implant body 22, and an actuator 23. In use, the processed signals transmitted by external antenna or coil 11 are received by the internal antenna or coil 21 and to provide control signals for actuator 23 to vibrate to generate the acoustic stimulation as required. The end 23b of the actuator 23 may be connected to any suitable part of the user's hearing system such as the stapes, or may in fact be connected directly to the oval window of the cochlea by way of a prosthetic stapes.

The outer material of the implant body 22 is biocompatible and provides a strong protective housing for the internal electronics. If the material has non-magnetic or has low electrical conductive properties on the operating FSK carrier frequency, then the internal antenna or coil 21 could even be integrated inside the implant body.

Figure 2 shows a broad method of processing an external signal (such as an acoustic signal or a test signal) for use in a medical implant system such as a DACS system, according to one aspect. At step 301, the external signal is received. At step 302, the received external signal is converted into an electrical signal. At step 303, the electrical signal is modulated using pulse modulation to provide a pulse modulated signal. As will be understood by the person skilled in the art, pulse modulation involves the modulation of pulses of a pulse signal with a modulating signal (such as the electrical signal). Various forms of pulse modulation may be used, including but not limited to, Pulse Amplitude Modulation (PAM), Pulse Width Modulation (PWM), Pulse position Modulation (PPM), Pulse Code Modulation (PCM), Differential PCM (DPCM), Adaptive DPCM (ADPCM), Delta Modulation, Sigma-Delta Modulation and Pulse Density Modulation (PDM).

At step 304, the pulse modulated signal is then modulated using digital modulation to provide a transmission signal which may then, in one example, be transmitted to an internal module of the medical implant system. Again as will be understood by the person skilled in the art, various forms of digital modulation may be used, including but not limited to, Frequency Shift Keying (FSK), Amplitude Shift Keying (ASK), On-Off Keying (OOK), Phase Shift Keying (PSK), Quadrature Amplitude Modulation (QAM), Minimum Shift Keying (MSK), Continuous Phase Modulation (CPM), and Pulse Position Modulation (PPM).

Figure 3 shows a broad arrangement for one implementation of the method described above by external module 10 of a medical implant system such as a DACS system. Shown there is external module 10, comprising an input such as an acoustic transducer such as a microphone 12, for receiving an acoustic signal such as sound from the surrounding environment. Alternatively, or in combination, input 12 may be a test button or other user interface that the user or an operator may use to generate a test or other signal. In the case where the input is an acoustic transducer, the transducer 12 converts the acoustic signal into an electrical signal. Connected to the transducer 12 is a first modulator, in this case a pulse modulator 13, which in use, receives as an input, the electrical signal from transducer 12. Pulse modulator 13 modulates the received electrical signal to provide a pulse modulated signal. As described above, various types of pulse modulators may be used, including but not limited to, Pulse Amplitude Modulator (PAM), Pulse Width Modulator (PWM), Pulse Position Modulator (PPM), Pulse Code Modulator (PCM), Differential PCM (DPCM), Adaptive DPCM (ADPCM), Delta Modulator, Sigma-Delta Modulator and Pulse Density Modulator (PDM).

Following the pulse modulator 13 is a digital modulator 14, which modulates the pulse modulated signal provided by pulse modulator 13. Digital modulator 14 modulates the pulse modulated signal to provide a transmission signal for subsequent transmission to the internal module 20 via antenna 11 as will be described in more detail below. Again as will be understood by the person skilled in the art, various types of digital modulators may be used, including but not limited to, Frequency Shift Keying (FSK) modulator, Amplitude Shift Keying (ASK) modulator, On-Off Keying (OOK) modulator, Phase Shift Keying (PSK) modulators, Quadrature Amplitude Modulation (QAM) modulator, Minimum Shift Keying (MSK) modulator, Continuous Phase Modulation (CPM) modulator, and Pulse Position Modulation (PPM) modulator.

Figure 4 shows a broad method of providing a control signal to an actuator of an implanted medical device. In step 401, a transmission signal is received. The transmission signal comprises a pulse modulated electrical signal which is further modulated using digital modulation. Such a transmission signal may be generated by the method and apparatus as described above with reference to Figures 2 and 3 for example. In step 402, the received transmission signal is digitally demodulated to remove the digital demodulation. The type of digital demodulation used would be appropriate to the type of digital modulation used, and may be any suitable including those described above.

In step 403, the digitally demodulated signal is then applied to the input of an amplifier to then provide the control signal for use with an actuator of for example, a DACS system.

Figure 5 shows one possible implementation of an internal module for carrying out the method described above. Shown in Figure 5 is internal module 20 comprising a receiver 21, such as an internal antenna or internal coil for receiving a transmission signal, a digital demodulator 25 for digitally demodulating the transmission signal to provide the digitally demodulated signal, and an amplifier 26, an input of which is connected to the output of digital demodulator 25. The output of amplifier 26 provides the control signal which may be applied to an actuator.

In one form, the digital demodulator 25 is an FSK demodulator. In one form, the amplifier 26 is a Class D amplifier. In one form, the internal module 20 also comprises the actuator 23.

Figure 6 shows broad arrangement of a medical implant system such as a DACS system 100 incorporating the external module 10 of Figure 3 and the internal module 20 of Figure 5 described above. In this example, there is shown external module 10 with input 12 for receiving an external signal, pulse modulator 13 for pulse modulating an electrical signal provided by input 12, digital modulator 14 for digitally modulating the pulse modulated signal from pulse modulator 13 to provide the transmission signal and external antenna 11 for transmitting the transmission signal transcutaneously through tissue 50 of the user.

Shown implanted within the user, is internal module 20. Internal module 20 comprises internal antenna 21 for receiving the transmission signal transmitted transcutaneously by external antenna 11, digital demodulator 25 for removing the digital demodulation from the transmission signal, and amplifier 26, for receiving the digitally demodulated signal from digital demodulator 25 and providing the control signal for use in controlling the actuator. Figure 6 shows that in one example. The internal module 20 may also comprise actuator 23, but in other example or embodiments, actuator 23 may be provided separately and later connected to internal module 20.

Figure 7 shows a simplified system block diagram of a medical implant system, a transcutaneous electromechanical stimulator system 100 such as a DACS system or a T-BAHA system, showing practical implementations and modifications of the external module 10 of Figure 3 and internal module 20 of Figure 4 and the system of Figure 6 described above. The two modules are separated by a layer of tissue 50 such as a portion of the user's scalp.

External module 10 includes an acoustic transducer 12 such as a microphone. The microphone 12 receives acoustic energy from the surrounding environment and translates this energy into electrical signals. These electrical signals are then input to a first modulator 13, in this case, a pulse modulator, to provide a pulse-modulated signal. The pulse-modulated signal is then applied to the input of a second modulator 14, in this case a digital modulator 14 to provide a further modulated signal or transmission signal. The transmission signal is then amplified in RF driver 15 and then applied to an antenna or coil 11 for wireless transmission through the layer of tissue 50 to the internal, implanted module 20. The internal module 10 may also include an audio pre-processing block (not shown) improving or optimizing the audio signal quality prior to modulation.

The internal module 20 includes internal antenna 21 as a receiving antenna or coil, for receiving the transmission signal from the transmitting antenna or coil 11. In this embodiment, the received signal is then applied to a power and modulation extracting block 24, which extracts power from the received transmission signal for powering a demodulator 25 and driver/amplifier 26 of the internal module 20. An additional post-processing circuitry (not shown) may also be included in the implant body 22. The demodulated and amplified signal is applied to the actuator 23 referred to above.

In one embodiment, the pulse modulator 13 is a Pulse Width Modulation (PWM) modulator and the digital modulator 14 is a Frequency Shift Keying (FSK) modulator. In another embodiment, the pulse modulator 13 is a Pulse Density Modulation (PDM) Modulator.

Figure 8 shows a more detailed block diagram of a transcutaneous modulated power link including components in the internal module 20 for demodulating and processing the data components in the transmitted transmission signal as well as extracting the power component in the transmitted transmission signal.

In Figure 8, external module 10 includes acoustic transducer or microphone 12, audio or signal pre-processing block 17 for conditioning the electrical signals generated by microphone 12, and for applying the conditioned signal to Pulse Width Modulation (PWM) or Pulse Density Modulation (PDM) modulator 13, which generates a PWM or PDM signal. This signal is then applied to the input of a Frequency Shift Keying (FSK) modulator 14 to generate a further modulated signal as the transmission signal which is then applied to external antenna or coil 11 for wireless transmission through tissue 50 to the internal module 20.

Internal module 20 includes the internal or receive antenna or coil 21 which receives the wirelessly transmitted transmission signal from the external module 10 and produces an electrical signal for use by the internal module 20. This electrical signal is applied to the input of rectification system 24a, which extracts a power component from the electrical signal which may be used to provide power to one or more of the remaining components of the internal module 20. In one form, the extracted power signal may be stored on a power storage device 30 such as a capacitor or small battery.

Any suitable extraction circuit may be used as will be understood by the person skilled in the art. This includes a simple rectification circuit with one or more diodes. An example of another suitable power rectification circuit is shown in US Patent No. 6,240,318, previously incorporated by reference.

The received electrical signal in this example, is also processed to extract the control information or control signal to actuate the mechanical actuator 23. In this example, the received electrical signal is applied to the input of a Frequency Shift Keying (FSK) demodulator 25 which removes the FSK modulation applied in the external module 10. This FSK demodulated signal is then applied directly to a class D amplifier 26. The output of this is then applied to a low pass filter or integrator 28, the output of which is adapted or optimized to the impedance of the actuator by the amplifier 26 to load matching block 29. Depending on the type of actuator load the matching block 29 and low-pass filter 28 could be created by a single block with combined functionality e.g. a passive network of inductors and/or capacitors. The output of this is then applied to the mechanical actuator 23 which generates stimulating vibrations in accordance with the signals applied. A suitable mechanical actuator is described in International Patent Application No. PCT/AU2005/001801 (WO2006/058368) previously incorporated by reference.

In one example, the transcutaneous link emanating from the external device or module 10 is an FSK modulated signal that is derived from the PWM or PDM signal. Therefore the auditory signal emanating from a microphone or any other audio source is first transformed into a PWM or PDM signal and then connected to the input of the FSK modulator. The modulated FSK signal is placed on an antenna or coil powering the implant with a quasi-continuous envelope signal enabling a maximized power transfer from an external power source to the implant.

In one embodiment, the implant module contains a Class D amplifier 26 that can directly be driven by the FSK demodulator. This provides a significant simplification on the electrical circuit of the implant or internal module 20. Class D amplifiers have a theoretical efficiency of 100%. The output of the Class D amplifier 26 is connected to the actuator 23 via a suitable filter network (for example, RC or LC low pass filter, integrator) to block the PWM carrier and recover the original audio signal.

A further advantage of the arrangement of Figure 8 is that the FSK demodulator 25 of the implant device or internal module 20 is less susceptible to signal distortion caused by sinking the necessary implant power from the secondary or internal antenna or coil 21 (part of the implant resonance tank). Thus the transcutaneous FSK signal containing power and PWM or PDM signal is very robust against clipping which can be caused by non-linear elements and power rectification of the implant.

PWM compares the analogue audio input signal to a triangular or sawtooth shaped waveform at a fixed carrier frequency well above the audio range (e.g. 200KHz). The comparator's output gives a stream of pulses with variable width. The width of each pulse is proportional to the amplitude of the audio input signal. Figure 9 illustrates the creation of PWM and one embodiment of a PWM modulation circuitry is depicted in Figure 11.

Figure 10 shows a modification of the arrangement of Figure 8. In this embodiment, as in the embodiment of Figure 8, external module 10 includes acoustic transducer or microphone 12, audio or signal pre-processing block 17 for conditioning the electrical signals generated by microphone 12, and for applying the conditioned signal to Pulse Width Modulation (PWM) or Pulse Density Modulation (PDM) modulator 13, which generates a PWM or PDM signal. This signal is then applied to the input of a Frequency Shift Keying (FSK) modulator 14 to generate a further modulated signal or transmission signal which is then applied to antenna or coil 11 for wireless transmission through tissue 50 to the internal module 20.

In this modification, a fixed relationship or frequency ratio between the frequencies used by the PWM modulator and FSK modulator is provided as seen in Figure 10. This provides an improved signal to noise ratio of the demodulated signal in the internal module. This fixed relationship is in one form, equal to an integer ratio between the FSK carrier and PWM frequency. This can be accomplished by a divider or a phase locked loop (PLL) with phase comparator.

PDM is accomplished by a sigma-delta modulator giving a variable rate of pulses which is proportional to the amplitude of the audio input signal in a given time window. Each pulse has the same time duration.

In other examples, the FSK modulation may be replaced by other forms of modulation such as Continuous Phase Frequency Shift Keying (CPFSK), Phase Shift Keying (PSK), Amplitude Phase Shift Keying (ASK) or On Off Keying (OOK). The Class D amplifier 26 may still be driven directly by an OOK demodulator. Using an OOK modulation would still reduce the number of electrical components on the demodulator block of the implant in comparison with prior art arrangements. A simple OOK envelope detector could be made using a diode loaded to an RC parallel circuit.

In other examples, the Class D amplifier may be replaced by a Class G amplifier.

Figure 12 shows an application of the various aspects of the present invention to a Transcutaneous Bone Anchored Hearing Aid (T-BAHA) system 100. Shown in Figure 12 is external module or device 10 including microphone 12, audio filter and amplifier (signal conditioning) 17, PWM modulator 13, FSK modulator 14, radio frequency (RF) driver 15 and antenna 11.

The internal device or module 20 includes receive antenna 21, power rectifier circuit 24a, FSK demodulator 25 which is driving a Class D or Class G amplifier 26, which drives actuator 23. In this example, actuator 23 is a piezoelectric device.

Figure 12 shows a simplified form of the implant 20 containing an implant coil, a power rectification block (synchronous or diode half-wave/full wave rectification), an FSK demodulator, a Class D audio amplifier and an actuator. In this example, all components may be embedded in one or more biocompatible implant casings. There could also optionally be an implantable power source (e.g. battery) to be charged over the transcutaneous link here described. In this example the frequency sources or clocks used by the modulators of the external module 10 are derived from a common frequency source or clock unit 18.

In one embodiment, there is a fixed relationship between the frequencies generated by the PWM modulator and FSK modulator improving the signal to noise ratio of the demodulated signal in the internal module. The clock unit (18) could also contain frequency dividers, multipliers and PLLs, guaranteeing the fixed relationship between f_{clk1} and f_{clk2}. The circuit complexity remains in the external module, with a simplified arrangement of the internal module. When using an OOK modulator there could be used an OOK carrier frequency with a constant frequency relationship to the frequency source used to establish the PWM signal. In one embodiment, the frequency ratio between the frequency sources of the digital modulator and the pulse modulator is an integer (e.g.. 200KHz PWM and 5MHz OOK carrier).

Figure 13 shows a particular arrangement of another embodiment of back-end components in the internal module or device 20 of a DACS system. Shown here is Class D amplifier 26 with an LC low pass filter arrangement on its output, driving actuator 23. In this example, the end 23b of actuator 23 is connected to the stapes of the user's middle ear.

Figures 14 to 17 show different electrical schematic examples of the internal part or internal module 20.

Figure 14 shows a circuit diagram of an internal module 20 with FSK demodulation by a phase-locked loop (PLL) 25. PLL detectors are well known to the person skilled in the art.

Figure 15 shows a circuit diagram of an internal module 20 with FSK demodulation by slope detection. The LC tank (L2 and C4) is above or below the nominal FSK carrier RF frequency. Frequency variation on one sloping side of the LC tuning curve gives amplitude variation. This signal can be directly connected to a class D amplifier 26.

Figure 16 shows a circuit diagram of an internal module 20 with OOK demodulation using an envelope diode detector using a simple RC network (R1 and C3).

Figure 17 shows a circuit diagram of an internal part with OOK demodulation using an envelope diode detector optimized for 200KHz PWM carrier (L2, C3 resonance at 200KHz). A simple RC network may also be used. The OOK demodulated signal is connected directly to the Class-D amplifier 26. This amplifier consists of push-pull MOSFETs in half- or full bridge architecture. For the practical implementation of the Class-D amplifiers logic inverters may be used.

Figures 18 to 23 show a specific implementation of one embodiment using OOK modulation and demodulation. A 200kHz PWM scheme is used, over a 5MHz RF link. Figure 18 shows a block diagram of the major components of medical device system 100 with external module 10 and internal module 20. In this example, external module 10 comprises input or microphone 12, an audio pre-processing block 17, pulse modulator (PWM) block 13, digital modulator as an OOK modulator 14, a coil driver block 15 and external antenna or primary coil 11. Any suitable audio pre-processor 17 may be used and the workings of which are well known to the person skilled in the art and will not be described in any further detail. The external module 10 may be powered by an external battery providing for example, about 24mW, with about 3V operating voltage and about 8mA DC current.

Internal module 20 in this example, comprises internal antenna or secondary coil 21, power extraction block 24a, digital or OOK demodulator 25, Class D amplifier 26, impedance matching block 27 and piezo-electric actuator 23.

Figure 19 shows pulse modulator (PWM) block 13 in detail. The PWM is provided by a logic gate inverter (IC2) together with the capacitor network and switches SW1 to SW6 as shown. Switches SW5 and SW6 are normally closed. The coarse PWM frequency is set by selection of SW1 to SW4. The generated output is a triangular wave of about 800mV peak to peak. This triangular wave is provided as an input to comparator IC1 (for example TLC3702ID). Applied to the inverting input of IC1 is the processed audio signal. The output of comparator IC1 is a PWM wave of about 3V peak to peak. Any ripple or noise on the 3V power supply is filtered out by placement of 22uF and 220nF capacitors on Vdd (voltage supply pin) of IC1 and IC2.

Figure 20 shows an example circuit for digital modulator 14. This circuit uses two inverter logic gates (74AC04) to generate an OOK or FSK signal modulated signal at 4.5 to 6.5MHz frequency, The output is buffered by two additional inverters placed in parallel. The RC oscillator of the modulator is formed by a resistor R=10K, two capacitors C=100pF in parallel with 7-100pF and a single inverter (pin 13/12). In the case of an OOK modulator SW1 is closed and SW2 opened. When the diode MCL4148 is not conducting or reversely polarized the oscillator will start-up and becomes operational. This happens when zero volt is applied at the input of J1 (pin 2 of IC1 is +3Volts and pin 13 is about +1.5V). In this case 3V is applied at the input of J1 the diode starts to conduct on the RC oscillator will stop.

The input and output of the OOK modulator 14 are shown in Figure 21. The upper trace shows the input signal at J1 and the lower trace shows the output signal at J2.

Figure 22 shows an example circuit for the coil driver 15 with a differential output The primary coil L is tuned to about 5MHz resonance by a series capacitor C (47pF in parallel with 7-100pF). The inverter gates of the differential output drivers are placed 2 by 2 in parallel to provide sufficient current going through the series resonant circuit LC. The coil driver 15 in this example uses a total of 6 inverter logic gates (74AC04).The 4 MCL4148 diodes protect the circuit from high transients caused by the LC tank or ESD.

Turning now to the details of the internal module or implant 20, Figure 23 shows a basic example circuit for the implant. The received OOK transmission signal transmitted by external antenna or primary coil 11 is received by internal antenna or secondary coil 21, which in this example comprises an 8-turn copper coil of about 28mm diameter. Diode D2 provides the rectification to extract the power component from the transmission signal to provide power to the remainder of the implant circuit. The transmission signal is applied to the input of PWM demodulator provided by D1, switches SW1 and SW2 with respective resistors 680 or /and 1K2 and the resonance tank tuned to about 200kHz. D1 and D2 are e.g.. standard Si-diodes such as MCL4148.

The output of this block is applied to the Class D amplifier 26 provided by the 74AC04 logic inverters, with a 2 to 6 volt range. The Class D output is differential.
The additional 470uH inductor is provided between the output of amplifier 26 and the piezo-electric transducer T1 providing the actuator 23 to limit the current at 250kHz. Additional zener diodes or transorbs could be used to protect the circuit from over-voltages (not shown).

The various aspects of the present invention provide several advantages over current systems. For example, the arrangement shown allows much of the circuit complexity to remain in the external module 10, with a simplified arrangement of the internal module 20. The internal circuitry is simplified in one form by having the demodulator directly driving the amplifier. Furthermore, the arrangement does not require a separate PWM or PDM demodulator to remove the Pulse Width Modulation or Pulse Density Modulation of the original audio signal applied in the external module. The arrangements described herein may be used in a uni-directional system (i.e. power and data flow from the external module to the internal module) thus allowing for further simplification of the internal module.

The various aspects of the present invention have been described with reference to specific embodiments.

Throughout the specification and the claims that follow, unless the context requires otherwise, the words "comprise" and "include" and variations such as "comprising" and "including" will be understood to imply the inclusion of a stated integer or group of integers, but not the exclusion of any other integer or group of integers.

The reference to any prior art in this specification is not, and should not be taken as, an acknowledgement of any form of suggestion that such prior art forms part of the common general knowledge.

## Claims

1. A method of providing a control signal for application to a mechanical actuator (23) of an
implantable hearing device (100), the method comprising:
receiving an audio signal (301);
converting the audio signal (302) into an electrical signal;
modulating the electrical signal (303) using pulse modulation to provide a pulse modulated signal;
modulating the pulse modulated signal (304) using a digital modulation to provide a transmission signal for transmitting to an internal module (20) of the implanted hearing device (100);
receiving the transmission signal (401);
digitally demodulating the received transmission signal (402) to remove the digital modulation; and
applying the demodulated signal (403) to an input of an amplifier (26) to
provide the control signal for application to the mechanical actuator (23).

2. A method as claimed in claim 1 wherein the pulse modulation is at least one of Pulse Width Modulation (PWM) and Pulse Density Modulation (PDM), and the digital modulation is at least one of Frequency Shift Keying (FSK), Continuous Phase Frequency Shift Keying (CPFSK), Phase Shift Keying (PSK, and On Off Keying (OOK).

3. A method as claimed in any one of the preceding claims wherein there is provided a frequency ratio between frequencies used in the pulse width modulation and the digital modulation, wherein the frequency ratio is an integer.

4. A method as claimed in any one of the preceding claims wherein the amplifier (26) is a Class D amplifier.

5. A method as claimed in any one of the preceding claims further comprising extracting a power component from the received transmission signal.

6. An internal module (20) for a hearing implant system (100) comprising:
a mechanical actuator (23);
a receiver (21) for receiving a transmission signal comprising a pulse modulated electrical signal further modulated using digital modulation;
a digital demodulator (25) for digitally demodulating the received transmission signal; and
an amplifier (26) for receiving the digitally demodulated signal and for providing a control signal to the mechanical actuator (23).

7. An internal module (20) as claimed in claim 6 wherein the digital demodulator (25) is an FSK demodulator.

8. An internal module (20) as claimed in any one of claims 6 - 7 wherein the amplifier (26) is a Class D amplifier.

9. An internal module (20) as claimed in any one of claims 6 - 8 further comprising a power extracting circuit (24) for extracting a power component from the received transmission signal.

10. A Direct Acoustic Cochlear Stimulator system (100) comprising:
an external module (10) for processing an external signal; and
an internal module (20) as recited by any one of claims 6 - 9,
wherein the external module (10) is configured for transmitting the processed external signal transcutaneously to the implanted internal module (20); and
wherein the external module (10) comprises:
an input (12) for receiving the external signal and for converting the received external signal into an electrical signal;
a pulse modulator (13) for modulating the electrical signal to provide a pulse modulated signal;
a digital modulator (14) for modulating the pulse modulated signal to provide a transmission signal; and
an antenna (11) for transmitting the transmission signal.

11. A Direct Acoustic Cochlear Stimulator system (100) as claimed in claim 10 wherein the pulse modulator (13) of the external module (10) is at least one of a Pulse Width Modulation (PWM) modulator and a Pulse Density Modulation (PDM) modulator, and the digital modulator (14) of the external module (10) is at least one of a Frequency Shift Keying (FSK) modulator, a Continuous Phase Frequency Shift Keying (CPFSK) modulator, a Phase Shift Keying (PSK) modulator, and a On Off Keying (OOK) modulator.

12. A Direct Acoustic Cochlear Stimulator system (100) as claimed in claim 10 wherein the pulse modulator (13) and the digital modulator (14) of the external module (10) are related to each other by a fixed frequency ratio.

13. A Direct Acoustic Cochlear Stimulator system (100) as claimed in claim 12 wherein the fixed frequency ratio is an integer.

## Patentansprüche

1. Verfahren zur Bereitstellung eines Steuersignals für Zuleitung zu einem mechanischen Aktuator (23) eines implantierbaren Hörgeräts (100), wobei das Verfahren umfasst:
Empfangen eines akustischen Signals (301);
Umwandeln des akustischen Signals (302) in ein elektrisches Signal;
Modulieren des elektrischen Signals (303) unter Anwendung von Pulsmodulation zur Bereitstellung eines pulsmodulierten Signals;
Modulieren des pulsmodulierten Signals (304) unter Anwendung einer digitalen Modulation, um ein Übertragungssignal zur Übertragung zu einem inneren Modul (20) des implantierten Hörgeräts (100) bereitzustellen;
Empfangen des Übertragungssignals (401);
digitales Demodulieren des empfangenen Übertragungssignals (402), um die digitale Modulation zu entfernen; und
Zuführen des die modulierten Signals (403) zu einem Eingang eines Verstärkers (26), um das Steuersignal für die Zuleitung zu dem mechanischen Aktuator (23) bereitzustellen.

2. Verfahren nach Anspruch 1, wobei die Pulsmodulation eine Pulsweitenmodulation (PWM) und/oder eine Pulsdichtemodulation (PDM) ist, und wobei die digitale Modulation eine Frequenzsprungmodulation (FSK) und/oder Frequenzsprungmodulation mit kontinuierlicher Phase (CPFSK) und/oder eine Phasenumtastung (PSK) und/oder eine EIN-AUS-Umtastung (OOK) ist.

3. Verfahren nach einem der vorhergehenden Ansprüche, wobei ein Frequenzverhältnis zwischen Frequenzen, die in der Pulsweitenmodulation und der digitalen Modulation verwendet sind, bereitgestellt wird, und wobei das Frequenzverhältnis eine ganze Zahl ist.

4. Verfahren nach einem der vorhergehenden Ansprüche, wobei der Verstärker (26) ein Klasse-D-Verstärker ist.

5. Verfahren nach einem der vorhergehenden Ansprüche, das ferner Extrahieren einer Leistungskomponente aus dem empfangenen Übertragungssignal umfasst.

6. Inneres Modul (20) für ein Implantat-Hörsystem (100), mit:
einem mechanischen Aktuator (23);
einem Empfänger (21) zum Empfangen eines Übertragungssignals, das ein pulsmoduliertes elektrisches Signal enthält, das weiter unter Anwendung digitaler Modulation moduliert ist;
einem digitalen Demodulator (25) zum digitalen Demodulieren des empfangenen Übertragungssignals; und
einem Verstärker (26) zum Empfangen des digital demodulierten Signals und zur Zuführung eines Steuersignals zu dem mechanischen Aktuator (23).

7. Inneres Modul (20) nach Anspruch 6, wobei der digitale Demodulator (25) ein FSK-Demodulator ist.

8. Inneres Modul (20) nach einem der Ansprüche 6-7, wobei der Verstärker (26) ein Klasse-D-Verstärker ist.

9. Inneres Modul (20) nach einem der Ansprüche 6-8, das ferner eine Leistungsextraktionsschaltung (24) zum Extrahieren einer Leistungskomponente aus dem empfangenen Übertragungssignal umfasst.

10. System zur direkten akustischen Stimulation der Innenohrschnecke (100), mit:
einem äußeren Modul (10) zur Verarbeitung eines externen Signals; und
einem inneren Modul (20) nach einem der Ansprüche 6-9,
wobei das äußere Modul (10) zur subkutanen Übertragung des verarbeiteten äußeren Signals zu dem implantierten inneren Modul (20) ausgebildet ist; und
wobei das äußeren Modul (10) umfasst:
einen Eingang (12) zum Empfangen des äußeren Signals und zur Umwandlung des empfangenen äußeren Signals in ein elektrisches Signal;
einen Pulsmodulator (13) zum Modulieren des elektrischen Signals, um ein pulsmoduliertes Signal bereitzustellen;
einen digitalen Modulator (14) zum Modulieren des pulsmodulierten Signals, um ein Übertragungssignal bereitzustellen; und
eine Antenne (11) zur Übertragung des Übertragungssignals.

11. System zur direkten akustischen Stimulation der Innenohrschnecke (100) nach Anspruch 10, wobei der Pulsmodulator (13) des äußeren Moduls (10) ein Pulsweitenmodulations-(PWM) Modulator und/oder ein Pulsdichtemodulations-(PDM) Modulator ist und wobei der digitale Modulator (14) des äußeren Moduls (10) ein Frequenzsprungmodulations-(FSK) Modulator und/oder ein Modulator mit Frequenzsprungmodulation mit kontinuierlicher Phase (CPFSK) und/oder ein Phasenumtastungs-(PSK) Modulator und/oder ein EIN-AUS-Umtastungs-(OOK) Modulator ist.

12. System zur direkten akustischen Stimulation der Innenohrschnecke (100) nach Anspruch 10, wobei der Pulsmodulator (13) und der digitale Modulator (14) des äußeren Moduls (10) über ein festgelegtes Frequenzverhältnis miteinander in Beziehung stehen.

13. System zur direkten akustischen Stimulation der Innenohrschnecke (100) nach Anspruch 12, wobei das festgelegte Frequenzverhältnis eine ganze Zahl ist.

## Revendications

1. Procédé de délivrance d'un signal de commande en vue d'une application sur un actionneur mécanique (23) appartenant à un dispositif d'écoute implantable (100), le procédé comprenant :
la réception d'un signal audio (301),
la conversion du signal audio (302) en un signal électrique,
la modulation du signal électrique (303) en utilisant une modulation d'impulsion pour fournir un signal modulé par impulsion,
la modulation du signal modulé par impulsion (304) en utilisant une modulation numérique pour fournir un signal de transmission destiné à être émis vers un module interne (20) du dispositif d'écoute implanté (100),
la réception du signal de transmission (401),
la démodulation numérique du signal de transmission reçu (402) afin d'éliminer la modulation numérique, et
l'application du signal démodulé (403) à une entrée d'amplificateur (26) afin de fournir le signal de commande en vue d'une application à l'actionneur mécanique (23).

2. Procédé selon la revendication 1, dans lequel la modulation d'impulsion est au moins l'une d'une modulation de largeur d'impulsion (PWM) et d'une modulation de densité d'impulsion (PDM), et la modulation numérique est au moins l'une d'une modulation par déplacement de fréquence (FSK), d'une modulation par déplacement de fréquence en phase continue (CPFSK), d'une modulation par déplacement de phase (PSK) et d'une modulation tout ou rien (OOK).

3. Procédé selon l'une quelconque des revendications précédentes, dans lequel il est fourni un rapport de fréquences entre les fréquences utilisées dans la modulation de largeur d'impulsion et la modulation numérique, le rapport de fréquences étant un nombre entier.

4. Procédé selon l'une quelconque des revendications précédentes, dans lequel l'amplificateur (26) est un amplificateur de classe D.

5. Procédé selon l'une quelconque des revendications précédentes comprenant en outre l'extraction d'une composante de puissance du signal de transmission reçu.

6. Module interne (20) destiné à un système d'implant auditif (100) comprenant :
un actionneur mécanique (23),
un récepteur (21) destiné à recevoir un système de transmission comprenant le signal électrique modulé par impulsion, modulé en outre en utilisant une modulation numérique,
un démodulateur numérique (25) destiné à démoduler numériquement le signal de transmission reçu, et
un amplificateur (26) destiné à recevoir le signal démodulé numériquement et à fournir un signal de commande à l'actionneur mécanique (23).

7. Module interne (20) selon la revendication 6, dans lequel le démodulateur numérique (25) est un démodulateur FSK.

8. Module interne (20) selon l'une quelconque des revendications 6 et 7, dans lequel l'amplificateur (26) est un amplificateur de classe D.

9. Module interne (20) selon l'une quelconque des revendications 6 à 8, comprenant en outre un circuit d'extraction de puissance (24) destiné à extraire une composante de puissance du signal de transmission reçu.

10. Système de stimulation cochléaire acoustique directe (100) comprenant :
un module externe (10) destiné à traiter un signal externe, et
un module interne (20) conforme à l'une quelconque des revendications 6 à 9,
dans lequel le module externe (10) est configuré pour transmettre le signal externe traité de manière transcutanée au module interne implanté (20), et
dans lequel le module externe (10) comprend :
une entrée (12) destinée à recevoir le signal externe et à convertir le signal externe reçu en un signal électrique,
un modulateur d'impulsion (13) destiné à moduler le signal électrique pour fournir un signal modulé par impulsion,
un modulateur numérique (14) destiné à moduler le signal modulé par impulsion pour fournir un signal de transmission, et
une antenne (11) destinée à émettre le signal de transmission.

11. Système de stimulation cochléaire acoustique directe (120) selon la revendication 10, dans lequel le modulateur d'impulsion (13) du module externe (10) est au moins l'un d'un modulateur pour modulation de largeur d'impulsion (PWM) et d'un modulateur pour modulation de densité d'impulsion (PDM), et le modulateur numérique (14) du module externe (10) est au moins l'un d'un modulateur pour modulation par déplacement de fréquence (FSK), d'un modulateur pour modulation par déplacement de fréquence en phase continue (CPFSK), d'un modulateur pour modulation par déplacement de phase (PSK) et d'un modulateur pour modulation tout ou rien (OOK).

12. Système de stimulation cochléaire acoustique directe (120) selon la revendication 10, dans lequel le modulateur d'impulsion (13) et le modulateur numérique (14) du module externe (10) sont reliés l'un à l'autre par un rapport de fréquences fixe.

13. Système de stimulation cochléaire acoustique directe (120) selon la revendication 12, dans lequel le rapport de fréquences fixe est un nombre entier.
